# EUROPEAN PATENT SPECIFICATION

(11) **EP 0 623 112 B1**
(45) Date of publication and mention of the grant of the patent: **30.12.1998**
(21) Application number: 93901257.1
(22) Date of filing: 21.12.1992
(51) Int. Cl.: C07D 215/38, C07D 401/12, A61K 31/47, A61K 31/505

(54) **4-Phenyl-3-(heteroarylureido)-1,2-dihydro-2-oxoquinoline derivatives as antihypercholesterolemic and antiatherosclerotic agents**
4-Phenyl-3-(heteroarylureido)-1,2-dihydro-2-oxochinolin Derivate, als antihypercholesterolemische und antiatherosklerotische Mittel
Dérivés de 4-phenyl-(heteroarylureido)-1,2-dihydro-2-oxoquinoleine utilises comme agents antihypercholesterolemie et antiatherosclerose

(30) Priority: 23.01.1992 US 824639
(43) Date of publication of application: 09.11.1994
(73) Proprietor: PFIZER INC., New York, N.Y. 10017 (US)
(72) Inventor: HAMANAKA, Ernest, S., Gales Ferry, CT 06335 (US)
(74) Representative: Moore, James William, Dr.
(86) International application number: US9210886
(87) International publication number: WO9315058

(56) References cited:
- EP-A- 0 354 994
- EP-A- 0 421 456
- WO-A-91/04027
- WO-A-91/12249
- WO-A-92/19614

## Description

### Background of the Invention

The present invention relates to new 4-aryl-3-(heteroarylureido)-1,2-dihydro-2-oxo-quinoline derivatives, pharmaceutical compositions comprising such compounds, novel 3-(p-nitrobenzyloxycarbonylamino) quinoline intermediates used in the synthesis of such compounds and the use of such compounds to inhibit intestinal absorption of cholesterol, lower serum cholesterol and reverse the development of atherosclerosis. The compounds are inhibitors of acyl coenzyme A: cholesterol acyltransferase (ACAT).

Cholesterol that is consumed in the diet (dietary cholesterol) is absorbed as free cholesterol by the mucosal cells of the small intestine. It is then esterified by the enzyme ACAT, packaged into particles known as chylomicrons, and released into the bloodstream. Chylomicrons are particles into which dietary cholesterol is packaged and transported in the bloodstream. By inhibiting the action of ACAT, the compounds of this invention prevent intestinal absorption of dietary cholesterol and thus lower serum cholesterol levels. They are therefore useful in preventing atherosclerosis, heart attacks and strokes.

By inhibiting the action of ACAT, the compounds of the present invention also enable cholesterol to be removed from the walls of blood vessels. This activity renders such compounds useful in slowing or reversing the development of atherosclerosis as well as in preventing heart attacks and strokes.

Other inhibitors of ACAT are referred to in United states Patents 4,716,175 and 4,743,605 (a divisional of the '175 patent), the European Patent Applications having publication numbers 0 242 610, 0 245 687, 0 252 524, 0 354 994, and 0 418 071.

Certain ureas and thioureas as antiatherosclerosis agents are referred to in United States Patent 4,623,662 and in the European Patent Applications having publication numbers 0 335 374, 0 386 487, 0 370 740, 0 405 233 and 0 421 456.

### Summary of the Invention

The present invention relates to compounds of the formula 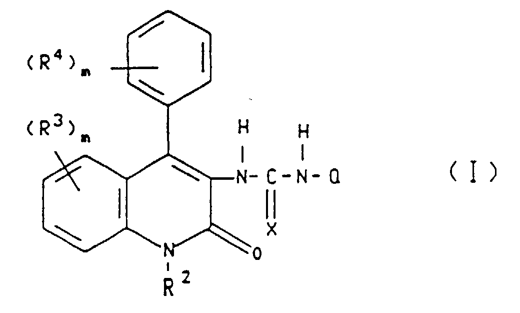 wherein each m is independently selected from 0 to 4;
R² is selected from hydrogen and (C₁-C₆) alkyl;
Each R³ and R⁴ is independently selected from halogen, (C₁-C₆) alkyl optionally substituted with one or more halogen atoms, (C₁-C₆) alkoxy optionally substituted with one or more halogen atoms, (C₁-C₆) alkylthio optionally substituted with one or more halogen atoms; nitro, carboxyl optionally esterified with a (C₁-C₆) alkyl group; hydroxyl, (C₁-C₄) acyloxy and (C₁-C₃) acyl;
X is sulfur or oxygen; and
Q is 6-(C₁-C₃) alkoxyquinolin-5-yl, 6-(C₁-C₃) alkylthioquinolin-5-yl, 6-(C₁-C₃)alkylquinolin-5-yl, 6-(C₁-C₃)alkylthioisoquinolin-5-yl, 6-(C₁-C₃)alkoxyisoquinolin-5-yl, 4,6-bis[(C₁-C₃)alkylthio]-2-methylpyrimidin-5-yl, 4,6-bis[(C₁-C₃)alkylthio]pyrimidin-5-yl, 2,4-bis[(C₁-C₃)alkylthio]-6-methylpyridin-3-yl or 2,4-bis[(C₁-C₃)alkylthio]pyridin-3-yl;
or a pharmaceutically acceptable salt of such compound.

Unless otherwise indicated, the term "halogen", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

Unless otherwise indicated, the term "one or more substituents" or "one or more halogen atoms", as used herein, refers to from one to the maximum number of substituents possible based on the number of available bonding sites.

The present invention also relates to compounds of the formula 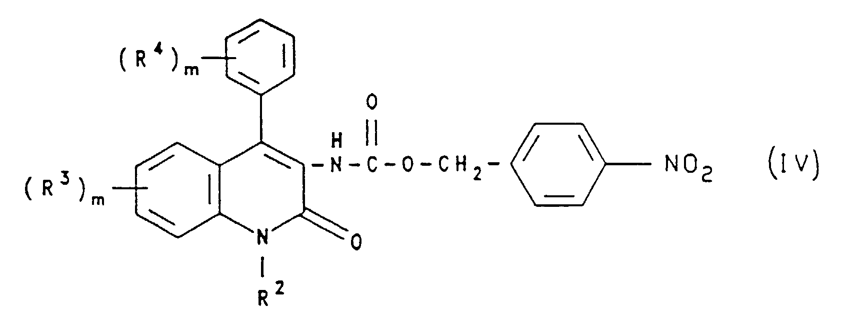 wherein m, R², R³ and R⁴ are as defined above. These compounds are useful as intermediates in the synthesis of compounds of the formula I.

Preferred compounds of the formula I are those wherein R² is methyl, m is at least one and R³ and R⁴ defined as above with the proviso that at least one of R³ is 6-halo or 6-alkyl and at least one of R⁴ is 2-halo.

More preferred compounds of the formula I are those wherein Q is 6-methoxyquinolin-5-yl, 6-methylthioquinolin-5-yl, 6-methoxyisoquinolin-5-yl, 6-methylthioisoquinolin-5-yl, 2-methyl-4,6-bis(methylthio)pyrimidin-5-yl, 6-methyl-2,4bis(methylthio)pyridin-3-yl, 2,4-bis(ethylthio)pyridin-3-yl, and 2,4-dimethoxy-6-methylpyridin-3-yl.

Other compounds of formula I include those wherein Q is 2,4,6-trimethylpyridin-3-yl, and 6-(4-methoxyphenylthio)quinolin-5-yl.

Specific preferred compounds of formula I include:
N-[2,4-Bis (methylthio)-6-methylpyridin-3-yl]-N'-[6-chloro-4-(2-chlorophenyl)-1,2-dihydro-1-methyl-2-oxoquinolin-3-yl]urea;
N-[4,6-Bis(methylthio)2-methylpyrimidin-5-yl]-N'-[6-chloro-4-(2-chlorophenyl)-1,2-dihydro-1-methyl-2-oxoquinolin-3-yl]urea;
N-[6-Chloro-4-(2-chlorophenyl)-1,2-dihydro-1-methyl-2-oxoquinolin-3-yl]-N'-(6-methylthioquinolin-5-yl)urea;
N-[6-Chloro-4-(2-chlorophenyl)-1,2-dihydro-1-methyl-2-oxoquinolin-3-yl]-N'-(6-methoxyquinolin-5-yl)urea;
N-[4,6-Bis (methylthio)-2-methylpyrimidin-5-yl]-N'-[4-(2-chlorophenyl)-1,2-dihydro-6-ethyl-1-methyl-2-oxoquinolin-3-yl]urea;
N-[2,4-Bis(methylthio)-6-methylpyridin-3-yl]-N'-[4-(2-chlorophenyl)-1,2-dihydro-6-ethyl-1-methyl-2-oxoquinolin-3-yl]urea;
N-4-(2-Chlorophenyl)-1,2-dihydro-6-ethyl-1-methyl-2-oxoquinolin-3-yl]-N'-(6-methylthioquinolin-5-yl)urea;
N-[4-(2-Chlorophenyl)-1,2-dihydro-6-ethyl-1-methyl-2-oxoquinolin-3-yl]-N'-(6-methoxyquinolin-5-yl]urea;
N-[2,4-Bis(methylthio)-6-methylpyridin-3-yl]-N'-[4-(2-chlorophenyl)-1,2-dihydro-6-isopropyl-1-methyl-2-oxoquinolin-3-yl]urea;
N-[4,6-Bis(methylthio)-2-methylpyrimidin-5-yl]-N'-[4-(2-chlorophenyl)-1,2-dihydro-6-isopropyl-1-methyl-2-oxoquinolin-3-yl]urea;
N-[4-(2-Chlorophenyl)-1,2-dihydro-6-isopropyl-1-methyl-2-oxoquinolin-3-yl]-N'-(6-methylthioquinolin-5-yl)urea;
N-[4-(2-Chlorophenyl)-1,2-dihydro-6-isopropyl-1-methyl-2-oxoquinolin-3-yl]-N'-(6-methoxyquinolin-5-yl)urea;
N-[2,4-Bis(methylthio)-6-methylpyridin-3-yl]-N'-[4-(2-chlorophenyl)-1,2-dihydro-1,6-dimethyl-2-oxoquinolin-3-yl]urea;
N-[4-(2-Chlorophenyl)-1,2-dihydro-1,6-dimethyl-2-oxoquinolin-3-yl]-N'-(6-methylthioquinolin-5-yl)urea;
N-[4,6-Bis(methylthio)-2-methylpyrimidin-5-yl]-N'-[4-(2-chlorophenyl)-1,2-dihydro-1,6-dimethyl-2-oxoquinolin-3-yl]urea;
N-[4-(2-Chlorophenyl)-1,2-dihydro-1,6-dimethyl-2-oxoquinolin-3-yl]-N'-(6-methoxyquinolin-5-yl)urea;
N-[6-Chloro-4-(2-chlorophenyl)-1,2-dihydro-1-methyl-2-oxoquinolin-3-yl]-N'-(6-dimethylaminoquinolin-5-yl)urea;
N-[6-Chloro-4-(2-chlorophenyl)-1,2-dihydro-1-methyl-2-oxoquinolin-3-yl]-N'-(2-dimethylamino-6-methyl-4-methylthiopyridin-3-yl)urea;
N-[6-Chloro-4-(2-chlorophenyl)-1,2-dihydro-1-methyl-2-oxoquinolin-3-yl]-N'-(6-methoxyisoquinolin-5-yl)urea.

The present invention also relates to a pharmaceutical composition for inhibiting ACAT, inhibiting intestinal absorption of cholesterol, reversing or slowing the development of atherosclerosis, or lowering the concentration of serum cholesterol in a mammal, including a human, comprising an amount of a compound of the formula I, or a pharmaceutically acceptable salt thereof, effective in inhibiting ACAT, inhibiting intestinal absorption of cholesterol, reversing or slowing the development of atherosclerosis, or lowering the concentration of serum cholesterol, and a pharmaceutically acceptable carrier.

The present invention also relates to a compound of formula (I), or a pharmaceutically acceptable salt thereof, or a pharmaceutical composition containing either entity, for use as a medicament.

The present invention further relates to the use of a compound of formula (I), or a pharmaceutically acceptable salt thereof, or a pharmaceutical composition containing either entity, for the manufacture of a medicament for inhibiting acyl coenzyme A: cholesterol acyltransferase or intestinal absorption of cholesterol, or for lowering the concentration of serum cholesterol, or for reversing or slowing the development of atherosclerosis.

Examples of pharmaceutically acceptable acid addition salts of the compounds of formula I salts are the salts of hydrochloric acid, p-toluenesulfonic acid, fumaric acid, citric acid, succinic acid, salicyclic acid, oxalic acid, hydrobromic acid, phosphoric acid, methanesulfonic acid, tartaric acid, di-p-toluoyl tartaric acid, and mandelic acid.

### Detailed Description of the Invention

Reaction scheme 1 below illustrates the synthesis of the compounds of this invention. Except where otherwise stated, R², R³, R⁴, Q, and m in the reaction scheme and discussion that follows are defined as above.

### Scheme 1

Scheme 1 illustrates the preparation of compounds of formula I. The starting materials II and III may be prepared by known methods (see, e.g., Ikeda, et al., European Patent Application 0 421 456 A2).

The compounds of formula III are then converted to the novel compounds of formula IV by reaction with p-nitrobenzyl alcohol in the presence of dibutyltin oxide.

The compounds of formula IV are reduced by hydrogen, in the presence of Raney nickel, to form the amines of formula V.

Reaction of the amino compounds of formula V with isocyanato group forming compounds, such as trichloromethyl chloroformate regenerates the compounds of formula III in a more highly purified state than when produced from the compounds of formula II.

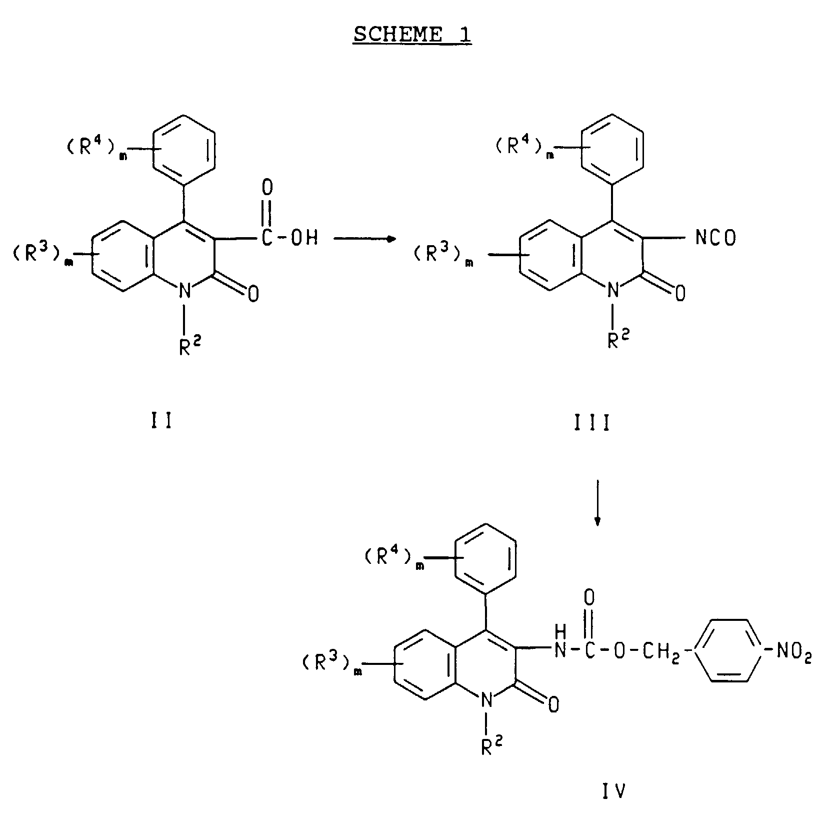

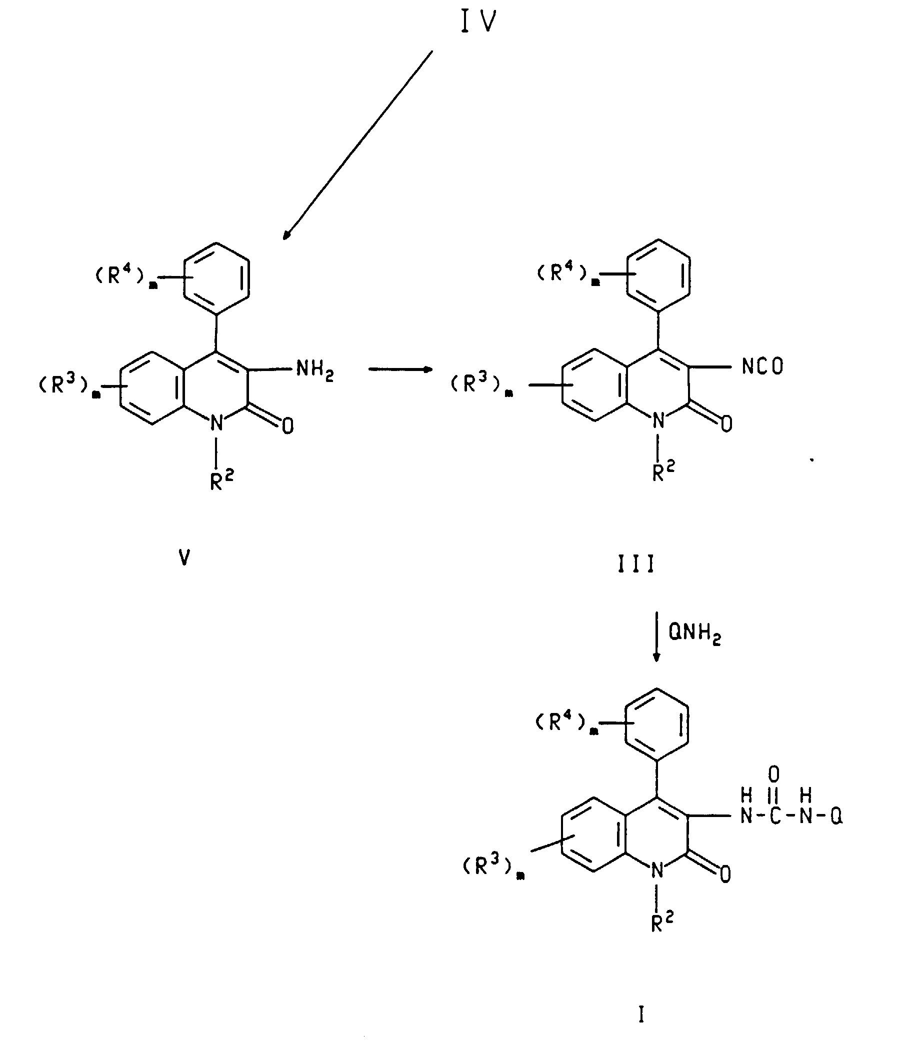

The compound of formula III, prepared as above, is then reacted with a compound of the formula QNH₂, wherein Q is defined as above, to form the compound of formula I.

The preparation of compounds of the formula QNH₂ is described in WO-A-92/19614.

In an alternative method the compound of formula I may be prepared by reacting a compound of the formula V with a compound of the formula QNC=X, whose preparation is disclosed in said application, wherein Q and X are as defined as above.

The novel compounds of formula I and the pharmaceutically acceptable salts thereof are useful as inhibitors of acyl coenzyme A: cholesterol acyltransferase (ACAT). As such they inhibit intestinal absorption of cholesterol in mammals and are useful in the treatment of high serum cholesterol in mammals, including humans. As used herein, treatment is meant to include both the prevention and alleviation of high serum cholesterol. The compound may be administered to a subject in need of treatment by a variety of conventional routes of administration, including orally, parenterally and topically. In general, these compounds will be administered orally or parenterally at dosages between 0.5 and 30 mg/kg body weight of the subject to be treated per day, preferably from 0.8 to 5 mg/kg. For an adult human of approximately 70 kg of body weight, the usual dosage would, therefore, be 3.5 to 2000 mg per day. However, some variation in dosage will necessarily occur depending on the condition of the subject being treated and the activity of the compound being employed. The person responsible for administration will, in any event, determine the appropriate dose for the individual subject.

A compound of formula I or a pharmaceutically acceptable salt thereof may be administered alone or in combination with pharmaceutically acceptable carriers, in either single or multiple doses. Suitable pharmaceutical carriers include inert solid diluents or fillers, sterile aqueous solution and various organic solvents. The resulting pharmaceutical compositions are then readily administered in a variety of dosage forms such as tablets, powders, lozenges, syrups, injectable solutions. These pharmaceutical compositions can, if desired, contain additional ingredients such as flavorings, binders, excipients. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of a compound of formula I or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. Such solutions are especially suitable for intravenous, intramuscular, subcutaneous and intraperitioneal administration. In this connection, the sterile aqueous media employed are all readily available by standard techniques known to those skilled in the art.

The activity of the compounds of the present invention as ACAT inhibitors may be determined by a number of standard biological or pharmacological tests. For example, the following procedure was used to determine the ACAT inhibiting activity of compounds of formula I. ACAT was assayed in microsomes isolated from chow fed Sprague-Dawley rats according to Bilheimer, J.T., Meth. Enzymol., 111, ps 286-293 (1985), with minor modifications. Microsomes from rat liver were prepared by differential centrifugation and washed with assay buffer prior to use. The assay mixture contained 25 ul of BSA (40 mg/ml), 30 ul of rat liver microsome solution (100 ug microsomal protein), 20 ul of assay buffer (0.1 M K₂PO₄, 1.0 mM reduced Glutathione, pH 7.4), 20 ug of cholesterol in 100 ul of a 0.6% Triton® WR-1339 solution in assay buffer, and 5 ul of test compound dissolved in 100% DMSO (total volume = 180 ul). The assay mixture was incubated for 30 min at 37°C. The reaction was started by the addition of 20 ul of ¹⁴C-Oleoyl-CoA (1000 uM, 2,000 dpm/nmol) and run for 15 min at 37°C. The reaction was stopped by the addition of 1 ml ETOH. The lipids were extracted into 4 ml hexane. A 3 ml aliquot was dried under N₂, and resuspended in 100 ul of chloroform. 50 ul of chloroform were spotted on a heat activated TLC plate and developed in hexane: diethyl ether: acetic acid (85:15:1, v:v:v). Incorporation of radioactivity into cholesteryl esters was quantified on a Berthold LB2842 Linear TLC Analyzer. ACAT inhibition was calculated relative to a DMSO control assay.

The activity of the compounds of formula I in inhibiting intestinal absorption of cholesterol may be determined by the procedure of Melchoir and Harwell, J. Lipid. Res., 26, 306-315 (1985).

The present invention is illustrated by the following examples.

Melting points are uncorrected. Proton nuclear magnetic resonance spectra (¹H NMR) and ¹³C nuclear magnetic resonance spectra (¹³C NMR) were measured for solutions in deuterochoroform (CDCl₃) or D₆-dimethylsulfoxide (DMSO-D₆) and peak positions are expressed in parts per million (ppm) downfield from tetramethylsilane (TMS). The peak shapes are denoted as follows: s, singlet; d, doublet; t, triplet; q, quartet; m, multiplet; b, broad; c, complex; h, heptet.

### PREPARATION 1

### 6-Chloro-4-(2-chlorophenyl)-1,2-dihydro-1-methyl-3-(4-nitrobenzyl)oxycarbonylamino)-2-oxoquinoline

Triethylamine (0.7 ml, 5 mmol) was added dropwise with stirring at room temperature to a solution of 6-chloro-4-(2-chlorophenyl)-1,2-dihydro-1-methyl-2-oxoquinoline-3-carboxylic acid (1.75 g, 5 mmol) and diphenylphosphoryl azide (1.19 ml, 5.5 mmol) in 35 ml benzene. The mixture was stirred at room temperature for 15 minutes and then refluxed for 30 minutes to yield a solution of 6-chloro-4-(2-chlorophenyl)-1,2-dihydro-3-isocyanato-1-methyl-2-oxoquinoline. 4-Nitrobenzyl alcohol (2.31 g, 15.1 mmol) and dibutyltin oxide (35 mg) were added to the cooled reaction mixture which was then refluxed for 4 h. The reaction solution was cooled to room temperature, washed with water, dried (sodium sulfate) and concentrated in vacuo. The solid residue was purified by column chromatography on silica gel (650 g), eluting with 3:2 hexane/ethyl acetate to yield the title compound as an off-white solid (1.9 g, 76% yield).

¹H NMR (300 MHz, CDCl₃) δ 3.70 (s, 3H), 5.07 (s, 2H), 6.83 (s, 1H), 7.10 (d, 1H), 7.24-7.46 (c, 6H), 7.53 (c, 2H), 8.14 (d, 2H) .

In a similar manner, the following 1-methyl-3-(4-nitrobenzyloxycarbonylamino)-2-oxoquinoline derivatives were prepared:

### PREPARATION 2

### 4-(2-Chlorophenyl)-1,2-dihydro-6-ethyl-1-methyl-3-(4-nitrobenzyloxycarbonylamino)-2-oxoquinoline

91% yield

¹H NMR (300 MHz, CDCl₃) δ 1.15 (t, 3H), 2.59 (q, 2H); 3.84 (s, 3H), 5.08 (s, 2H), 6.72 (s, 1H), 6.92 (s, 1H), 7.22-7.47 (c, 7H), 7.53 (d, 1H), 8.14 (d, 2H).

### PREPARATION 3

### 4-(2-Chlorophenyl)-1,2-dihydro-6-isopropyl-1-methyl-3-(4-nitrobenzyloxycarbonylamino)-2-oxoquinoline

96% yield.

¹H NMR (300 MHz, CDCl₃) δ 1.16 (d, 3H), 1.17 (d, 3H), 2.85 (h, 1H), 3.84 (s, 3H), 5.08 (s, 2H), 6.74 (s, 1H), 6.96 (d, 1H), 7.25-7.56 (c, 8H), 8.14 (d, 2H).

### PREPARATION 4

### 4-(2-Chlorophenyl)-1,2-dihydro-1,6-dimethyl-3-(4-nitrobenzyloxycarbonylamino)-2-oxoquinoline

74% yield.

¹H NMR (300 MHz, CDCl₃) δ 2.30 (s, 3H), 3.84 (s, 3H), 5.08 (s, 2H), 6.70 (s, 1H), 6.90 (s, 1H), 7.27-7.44 (c, 7H), 7.54 (d, 1H), 8.14 (d, 2H).

### PREPARATION 5

### 3-Amino-6-chloro-4-(2-chlorophenyl)-1,2-dihydro-1-methyl-2-oxoquinoline

A mixture of 6-chloro-4-(2-chlorophenyl)-1,2-dihydro-1-methyl-3-(4-nitrobenzyloxycarbonylamino)-2-oxoquinoline (1.9 g, 3.8 mmol) and Raney nickel (1.9 g, 50% slurry in water) in 75 ml dioxane and 75 ml methanol was shaken under hydrogen at 345 KPa (50 psi) in a Parr hydrogenation apparatus for 3 h. The catalyst was filtered and the filtrate was concentrated to dryness in vacuo. The residue was dissolved in 100 ml ethyl acetate and the solution was washed with 60 ml brine. The ethyl acetate solution was then dried (sodium sulfate) and concentrated in vacuo to a solid (1.2 g) which was purified by column chromatography on silica gel (150 g), eluting with 7:3 hexane/ethyl acetate to yield the title compound as an off-white solid (950 mg, 79% yield).

¹H NMR (300 MHz, CDCl₃) δ 3.85 (s, 3H), 4.39 (b, 2H), 6.83 (d, 1H), 7.28 (c, 3H), 7.46 (c, 2H), 7.62 (c, 1H).

In a similar manner, the following 3-amino-1,2-dihydro-1-methyl-2-oxoquinoline derivatives were prepared:

### PREPARATION 6

### 3-Amino-4-(2-chlorophenyl)-1,2-dihydro-6-ethyl-1-methyl-2-oxoquinoline

80% yield.

¹H NMR (300 MHz, CDCl₃) δ 1.15 (t, 3H), 2.57 (q, 2H), 3.86 (s, 3H), 4.27 (b, 2H), 6.68 (s, 1H), 7.15-7.35 (c, 3H), 7.45 (m, 2H), 7.63 (m, 1H).

### PREPARATION 7

### 3-Amino-4-(2-chlorophenyl)-1,2-dihydro-6-isopropyl-1-methyl-2-oxoquinoline

89% yield.

¹H NMR (300 MHz, CDCl₃) δ 1.16 (d, 3H), 1.163 (d, 3H), 2.82 (h, 1H), 3.86 (s, 3H), 4.27 (b, 2H), 6.70 (d, 1H), 7.24 (m, 1H), 7.33 (m, 2H), 7.46 (m, 2H), 7.62 (m, 1H).

### PREPARATION 8

### 3-Amino-4-(2-chlorophenyl)-1,2-dihydro-1,6-dimethyl-2-oxoquinoline

97% yield.

¹H NMR (300 MHz, CDCl₃) δ 2.28 (s, 3H), 3.85 (s, 3H), 4.27 (b, 2H), 6.66 (s, 1H), 7.16 (q, 1H), 7.3 (m, 2H), 7.46 (m, 2H), 7.63 (m, 1H).

### EXAMPLE 1

### N-[2,4-Bis(methylthio)-6-methylpyridin-3-yl]-N'-[6-chloro-4-(2-chlorophenyl)-1,2-dihydro-1-methyl-2-oxoquinolin-3-yl]urea

A solution of a 3-amino-6-chloro-4-(2-chlorophenyl)-1,2-dihydro-1-methyl-2-oxoquinoline (200 mg, 0.63 mmol) and trichloromethyl chloroformate (0.053 ml, 0.31 mmol) in 5 ml dichloromethane was refluxed under nitrogen overnight. The reaction solution was cooled and concentrated in vacuo to a solid,6-chloro-4-(2-chlorophenyl)-1,2-dihydro-3-isocyanato-1-methyl-2-oxoquinoline (214 mg, 99% yield). The isocyanate (107 mg, 0.31 mmol) was dissolved in dimethylformamide (1 ml), 3-amino-2,4-bis(methylthio)-6-methylpyridine (62 mg, 0.31 mmol) was added and the resulting solution was heated overnight under nitrogen at 80°C. The reaction mixture was cooled to room temperature, diluted with 50 ml ethyl acetate and the resulting solution was washed with 3x25 ml water and 25 ml brine, dried (sodium sulfate) and concentrated in vacuo. The solid residue (130 mg) was purified by column chromatography on silica gel (75 g), eluting with 3:1 dichloromethane/ethyl acetate to yield the title compound as a white solid (70 mg, 41% yield).

¹H NMR (300 MHz, CDCl₃) δ 2.33 (s, 3H), 2.43 (s, 3H), 2.46 (s, 3H), 3.79 (s, 3H), 6.58 (s, 1H), 6.88 (b, 1H), 7.07 (d, 1H), 7.28-7.54 (c, 7H).

In a similar, the following (1,2-dihydro-1-methyl-2-oxoquinolin-3-yl)urea derivatives were prepared:

### EXAMPLE 2

### N-[4,6-Bis(methylthio)2-methylpyrimidin-5-yl]-N'-[6-chloro-4-(2-chlorophenyl)-1,2-dihydro-1-methyl-2-oxoquinolin-3-yl]urea

13% yield.

¹H NMR (300 MHz, CDCl₃) δ 2.43 (s, 6H), 2.57 (s, 3H), 3.80 (s, 3H), 6.97 (b, 1H), 7.09 (d, 1H), 7.3-7.58 (c, 7H).

### EXAMPLE 3

### N-[6-Chloro-4-(2-chlorophenyl)-1,2-dihydro-1-methyl-2-oxoquinolin-3-yl]-N'-(6-methylthioquinolin-5-yl)urea

20% yield.

¹H NMR (300 MHz, CDCl₃) δ 2.34 (s, 3H), 3.77 (s, 3H), 7.11 (d, 1H), 7.2-7.6 (c, 10H), 7.77 (b, 1H), 7.98 (d, 1H), 8.78 (m, 1H).

### EXAMPLE 4

### N-[6-Chloro-4-(2-chlorophenyl)-1,2-dihydro-1-methyl-2-oxoquinolin-3-yl]-N'-(6-methoxyquinolin-5-yl)urea

63% yield.

¹H NMR (300 MHz, CDCl₃) δ 3.77 (s, 3H), 3.79 (s, 3H), 7.0 (b, lH), 7.11 (d, 1H), 7.2-7.63 (c, 9H), 7.70 (d, 1H), 8.00 (d, 1H), 8.74 (m, 1H).

### EXAMPLE 5

### N-[4,6-Bis(methylthio)-2-methylpyrimidin-5-yl]-N'-[4-(2-chlorophenyl)-1,2-dihydro-6-ethyl-1-methyl-2-oxoquinolin-3-yl]urea

19% yield.

¹H NMR (300 MHz, CDCl₃) δ 1.14 (t, 3H), 2.41 (s, 6H), 2.56 (s, 3H), 2.58 (q, 2H), 3.81 (s, 3H), 6.91 (s and b, 2H), 7.28-7.51 (c, 6H), 7.52 (m, 1H).

### EXAMPLE 6

### N-[2,4-Bis(methylthio)-6-methylpyridin-3-yl]-N'-[4-(2-chlorophenyl)-1,2-dihydro-6-ethyl-1-methyl-2-oxoquinolin-3-yl]urea

19% yield.

¹H NMR (300 MHz, CDCl₃) δ 1.14 (t, 3H), 2.34 (s, 3H), 2.44 (s, 3H), 2.46 (s, 3H), 2.58 (q, 2H), 3.81 (s, 3H), 6.58 (s, 1H), 6.68 (b, 1H), 6.90 (s, 1H), 7.29-7.56 (c, 7H).

### EXAMPLE 7

### N-4-(2-Chlorophenyl)-1,2-dihydro-6-ethyl-1-methyl-2-oxoquinolin-3-yl]-N'-(6-methylthioquinolin-5-yl)urea

19% yield.

¹H NMR (300 MHz, CDCl₃) δ 1.14 (t, 3H), 2.34 (s, 3H), 2.58 (q, 2H), 3.80 (s, 3H), 6.93, (s, 1H), 7.18-7.6 (c, 10H), 7.85 (c, 1H), 7.97 (d, 1H), 8.78 (m, 1H).

### EXAMPLE 8

### N-[4-(2-Chlorophenyl)-1,2-dihydro-6-ethyl-1-methyl-2-oxoquinolin-3-yl]-N'-(6-methoxyquinolin-5-yl)urea

4% yield.

¹H NMR (300 MHz, CDCl₃) δ 1.15 (t, 3H), 2.59 (q, 2H), 3.79 (s, 3H), 3.81 (s, 3H), 6.94 (s, 1H), 7.02-7.61 (c, 10H), 7.76 (d, 1h), 7.99 (d, 1H), 8.73 (m, 1H).

### EXAMPLE 9

### N-[2,4-Bis(methylthio)-6-methylpyridin-3-yl]-N'-[4-(2-chlorophenyl)-1,2-dihydro-6-isopropyl-1-methyl-2-oxoquinolin-3-yl]urea

32% yield.

¹H NMR (300 MHz, CDCl₃) δ 1.15 (d, 3H), 1.16 (d, 3H), 2.35 (s, 3H), 2.45 (s, 3H), 2.46 (s, 3H), 2.84 (h, 1H), 3.81 (s, 3H), 6.59 (s, 1H), 6.64 (b, 1H), 6.93 (d, lH), 7.32-7.56 (c, 7H).

### EXAMPLE 10

### N-[4,6-Bis(methylthio-2-methylpyrimidin-5-yl]-N'-[4-(2-chlorophelyl)-1,2-dihydro-6-isopropyl-1-methyl-2-oxoquinolin-3-yl]urea

29% yield.

¹H NMR (300 MHz, CDCl₃) δ 1.15 (d, 3H), 1.16 (d, 3H), 2.41 (s, 6H), 2.56 (s, 3H), 2.84 (h, 1H), 3.81 (s, 3H), 6.95 (s and b, 2H), 7.34-7.58 (c, 7H).

### EXAMPLE 11

### N-[4-(2-Chlorophenyl)-1,2-dihydro-6-isopropyl-1-methyl -2-oxoquinolin-3-yl]-N'-(6-methylthioquinolin-5-yl)urea

33% yield.

¹H NMR (300 MHz, CDCl₃) δ 1.15 (d, 3H), 1.16 (d, 3H), 2.32 (s, 3H), 2.84 (h, 1H), 3.79 (s, 3H), 6.98 (s, 1H), 7.2-7.62 (c, 10H), 7.81 (c, 1H), 7.95 (d, 1H), 8.77 (c, 1H).

### EXAMPLE 12

### N-[4-(2-Chlorophenyl)-1,2-dihydro-6-isopropyl-1-methyl-2-oxoquinolin-3-yl]-N'-(6-methoxyquinolin-5-yl)urea

10% yield.

¹H NMR (300 MHz, CDCl₃) δ 1.15 (d, 3H), 1.16 (d, 3H), 2.85 (h, 1H), 3.80 (s, 3H), 3.81 (s, 3H), 6.98 (s, 1H), 7.0-7.64 (c, 10H), 7.75 (d, 1H), 8.00 (d, 1H), 8.74 (m, 1H).

### EXAMPLE 13

### N-[2,4-Bis(methylthio)-6-methylpyridin-3-yl]-N'-4-(2-chlorophenyl)-1,2-dihydro-1,6-dimethyl-2-oxoquinolin -3-yl]urea

25% yield.

¹H NMR (300 MHz, CDCl₃) δ 2.28 (s, 3H), 2.31 (s, 3H), 2.41 (s, 3H), 2.45 (s, 3H), 3.78 (s, 3h), 6.57 (s, 1H), 6.88 (s and b, 2H), 7.26-7.54 (c, 7H).

### EXAMPLE 14

### N-[4-(2-Chlorophenyl)-1,2-dihydro-1,6-dimethyl-2-oxoquinolin-3-yl]-N'-(6-methylthioquinolin-5-yl)urea

31% yield.

¹H NMR 300 MHz, CDCl₃) δ 2.27 (s, 3H), 2.29 (s, 3H), 3.77 (s; 3H), 6.93 (s, 1H), 7.17-7.62 (c, 10H), 7.78 (c, 1H), 7.92 (d, 1H), 8.74 (m, 1H).

### EXAMPLE 15

### N-[4,6-Bis(methylthiol-2-methylpyrimidin-5-yl]-N'-[4-(2-chlorophenyl)-1,2-dihydro-1,6-dimethyl-2-oxoquinolin -3-yl]urea

23% yield.

¹H NMR (300 MHz, CDCl₃) δ 2.29 (s, 3H), 2.42 (s, 6H), 2.57 (s, 3H), 3.81 (s, 3H), 6.8 (b, 1H), 6.88 (s, 1H), 7.28-7.56 (c, 7H).

### EXAMPLE 16

### N-[4-(2-Chlorophenyl)-1,2-dihydro-1,6-dimethyl-2-oxoquinolin-3-yl]-N'-(6-methoxyquinolin-5-yl)urea

13% yield.

¹H NMR (300 MHz, CDCl₃) δ 2.30 (s, 3H), 3.79 (s, 3H), 3.80 (s, 3H), 6.92 (s, 1H), 6.94-7.63 (c, 10H), 7.77 (d, 1H), 7.99 (d, 1H), 8.73 (m, 1H).

### EXAMPLE 17

### N-[6-Chloro-4-(2-chlorophenyl)-1,2-dihydro-1-methyl-2-oxoquinolin-3-yl]-N'-(6-methoxyisoquinolin-5-yl)urea

10% yield.

¹H NMR (300 MHz, CDCl₃) δ 3.79 (s, 3H), 3.84 (s, 3H), 7.1 (b, 1H), 7.13 -(d, 1H), 7.33 (2d, 2H), 7.44-7.64 (c, 7H), 7.87 (d, 1H), 8.31 (b, 1H), 9.1 (b, 1H).

## Claims

1. A compound of the formula 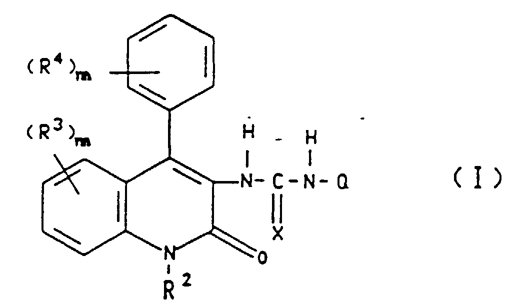 wherein each m is independently selected from 0 to 4;
R² is selected from hydrogen and (C₁-C₆) alkyl;
Each R³ and R⁴ is independently selected from halogen, (C₁-C₆) alkyl optionally substituted with one or more halogen atoms, (C₁-C₆) alkoxy optionally substituted with one or more halogen atoms, (C₁-C₆) alkylthio optionally substituted with one or more halogen atoms; nitro, carboxyl optionally esterified with a (C₁-C₆) alkyl group; hydroxyl, (C₁-C₄) acyloxy and (C₁-C₃) acyl;
X is sulfur or oxygen; and
Q is 6-(C₁-C₃)alkoxyquinolin-5-yl, 6-(C₁-C₃)alkylthioquinolin-5-yl, 6-(C₁-C₃) alkylquinolin-5-yl, 6-(C₁-C₃)alkylthioisoquinolin-5-yl, 6-(C₁-C₃)alkoxyisoquinolin-5-yl, 4,6-bis[(C₁-C₃)alkylthio]-2-methylpyrimidin-5-yl, 4,6-bis[(C₁-C₃)alkylthio]pyrimidin-5-yl, 2,4-bis[(C₁-C₃)alkylthio]-6-methylpyridin-3-yl or 2,4-bis[(C₁-C₃)alkylthio]pyridin-3-yl;
or a pharmaceutically acceptable salt thereof.

2. A compound according to claim 1, wherein R² is methyl, m is at least one and R³ and R⁴ are defined as above with the proviso that at least one of R³ is 6-halo or 6-alkyl and at least one of R⁴ is 2-halo.

3. A compound according to claim 1, wherein said compound is selected from the group consisting of
N-[2,4-Bis(methylthio)-6-methylpyridin-3-yl]-N'-[6-chloro-4-(2-chlorophenyl)-1,2-dihydro-1-methyl-2-oxoquinolin-3-yl]urea;
N-[4,6-Bis(methylthio)2-methylpyrimidin-5-yl]-N'-[6-chloro-4-(2-chlorophenyl)-1,2-dihydro-1-methyl-2-oxoquinolin-3-yl]urea;
N-[6-Chloro-4-(2-chlorophenyl)-1,2-dihydro-1-methyl-2-oxoquinolin-3-yl]-N'-(6-methylthioquinolin-5-yl)urea; and
N-[6-Chloro-4-(2-chlorophenyl)-1,2-dihydro-1-methyl-2-oxoquinolin-3-yl]-N'-(6-methoxyquinolin-5-yl)urea;
N-[4,6-Bis (methylthio)-2-methylpyrimidin-5-yl]-N'-[4-(2-chlorophenyl)-1,2-dihydro-6-ethyl-1-methyl-2-oxoquinolin-3-yl]urea;
N-[2,4-Bis(methylthio)-6-methylpyridin-3-yl]-N'-[4-(2-chlorophenyl)-1,2-dihydro-6-ethyl-1-methyl-2-oxoquinolin-3-yl]urea;
N-4-(2-Chlorophenyl)-1,2-dihydro-6-ethyl-1-methyl-2-oxoquinolin-3-yl]-N'-(6-methylthioquinolin-5-yl)urea;
N-[4-(2-Chlorophenyl)-1,2-dihydro-6-ethyl-1-methyl-2-oxoquinolin-3-yl]-N'-(6-methoxyquinolin-5-yl]urea;
N-[2,4-Bis(methylthio)-6-methylpyridin-3-yl]-N'-[4-(2-chlorophenyl)-1,2-dihydro-6-isopropyl-1-methyl-2-oxoquinolin-3-yl]urea;
N-[4,6-Bis(methylthio)-2-methylpyrimidin-5-yl]-N'-[4-(2-chlorophenyl)-1,2-dihydro-6-isopropyl-1-methyl-2-oxoquinolin-3-yl]urea;
N-[4-(2-Chlorophenyl)-1,2-dihydro-6-isopropyl-1-methyl-2-oxoquinolin-3-yl]-N'-(6-methylthioquinolin-5-yl)urea;
N-[4-(2-Chlorophenyl)-1,2-dihydro-6-isopropyl-1-methyl-2-oxoquinolin-3-yl]-N'-(6-methoxyquinolin-5-yl)urea;
N-[2,4-Bis(methylthio)-6-methylpyridin-3-yl]-N'-[4-(2-chlorophenyl)-1,2-dihydro-1,6-dimethyl-2-oxoquinolin-3-yl]urea;
N-[4-(2-Chlorophenyl)-1,2-dihydro-1,6-dimethyl-2-oxoquinolin-3-yl]-N'-(6-methylthioquinolin-5-yl)urea;
N-[4,6-Bis (methylthio)-2-methylpyrimidin-5-yl]-N'-[4-(2-chlorophenyl)-1,2-dihydro-1,6-dimethyl-2-oxoquinolin-3-yl]urea;
N-[4-(2-Chlorophenyl)-1,2-dihydro-1,6-dimethyl-2-oxoquinolin-3-yl]-N'-(6-methoxyquinolin-5-yl)urea;
N-[6-Chloro-4-(2-chlorophenyl)-1,2-dihydro-1-methyl-2-oxoquinolin-3-yl]-N'-(6-methoxyisoquinolin-5-yl)urea.

4. A compound according to claim 1 wherein Q is selected from the group consisting of 6-methoxyquinolin-5-yl, 6-methylthioquinolin-5-yl, 6-methoxyisoquinolin-5-yl, 6-methylthioisoquinolin-5-yl, 2-methyl-4,6-bis(methylthio)pyrimidin-5-yl, 6-methyl-2,4-bis(methylthio)pyridin-3-yl,2,4-bis(ethylthio)pyridin-3-yl,

5. A compound having the formula 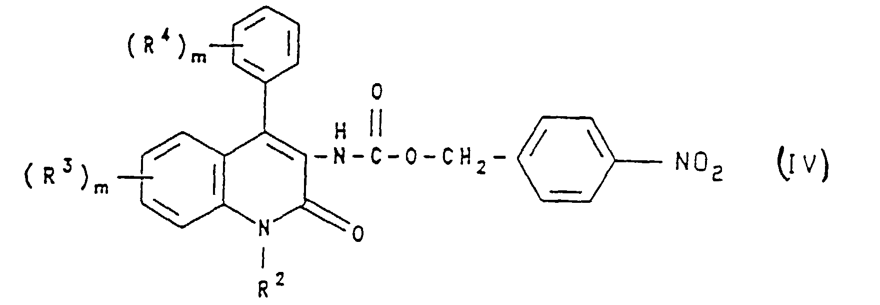 wherein m, R², R³ and R⁴ are defined as in claim 1.

6. A pharmaceutical composition comprising a compound of formula (I) or a pharmaceutically acceptable salt thereof according to any one of claims 1 to 4, together with a pharmaceutically acceptable diluent or carrier.

7. A compound of formula (I) or a pharmaceutically acceptable salt thereof according to any one of claims 1 to 4, or a pharmaceutical composition according to claim 6, for use as a medicament.

8. The use of a compound of formula (I) or a pharmaceutically acceptable salt thereof according to any one of claims 1 to 4, or a pharmaceutical composition according to claim 6, for the manufacture of a medicament for inhibiting acyl coenzyme A: cholesterol acyltransferase or intestinal absorption of cholesterol, or for lowering the concentration of serum cholesterol, or for reversing or slowing the development of atherosclerosis.

9. A process for the preparation of a compound of the formula I 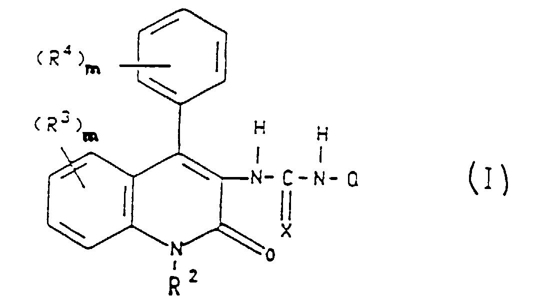 wherein R², R³, R⁴, X, m and Q are as previously defined in claim 1, which comprises either
(a) reaction of a compound of the formula III 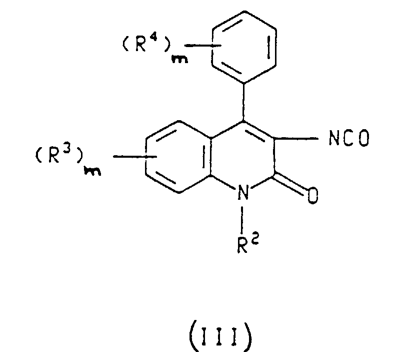 wherein R², R³, R⁴ and m are as previously defined in this claim, with a compound of the formula QNH₂ wherein Q is as previously defined in this claim, or
(b) reaction of a compound of the formula V 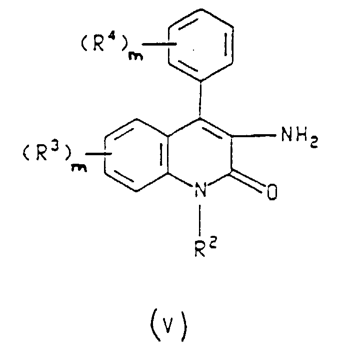 wherein R², R³, R⁴ and m are as previously defined in this claim, with a compound of the formula QN=C=X wherein Q and X are as previously defined in this claim, optionally followed by conversion of the product to a pharmaceutically acceptable salt.

## Patentansprüche

1. Verbindung der Formel 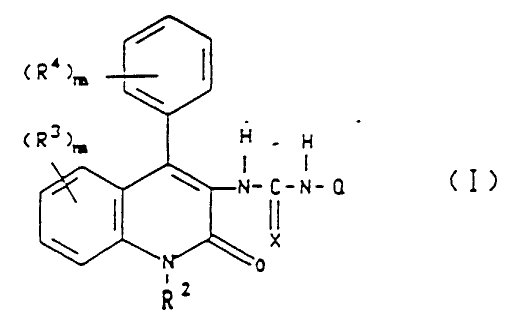 worin jedes m unhabhängig ausgewählt ist aus 0 bis 4;
R² ausgewählt ist aus Wasserstoff und (C₁-C₆)-Alkylresten;
jeder Rest R³ und R⁴ unabhängig ausgewählt ist aus Halogenatomen, (C₁-C₆) -Alkylresten, die gegebenenfalls mit einem oder mehreren Halogenatomen substituiert sind, (C₁-C₆)-Alkoxyresten, die gegebenenfalls mit einem oder mehreren Halogenatomen substituiert sind, (C₁-C₆)-Alkylthioresten, die gegebenenfalls mit einem oder mehreren Halogenatomen substituiert sind, Nitroresten, Carboxylresten, die gegebenenfalls mit einer (C₁-C₆)-Alkylgruppe verestert sind, Hydroxylresten (C₁-C₄)-Acyloxyresten und (C₁-C₃)-Acylresten;
X Schwefel oder Sauerstoff ist
Q ein 6-(C₁-C₃)-Alkoxychinolin-5-yl-, 6-(C₁-C₃)-Alkylthiochinolin-5-yl-, 6-(C₁-C₃)-Alkylchinolin-5-yl-, 6-(C₁-C₃)-Alkylthioisochinolin-5-yl-, 6-(C₁-C₃)-Alkoxy-isochinolin-5-yl-, 4,6-Bis-[(C₁-C₃)-alkylthio]-2-methylpyrimidin-5-yl-, 4, 6-Bis- [(C₁-C₃)-alkylthio]-pyrimidin-5-yl-, 2,4-Bis-[(C₁-C₃)-alkylthio]-6-methylpyridin-3-yl-oder 2,4-Bis-[(C₁-C₃)-alkylthio]-pyridin-3-ylrest ist
oder ein pharmazeutisch annehmbares Salz davon.

2. Verbindung nach Anspruch 1, worin R² ein Methylrest ist,
m mindestens 1 ist und
R³ und R⁴ wie oben definiert sind, mit dem Vorbehalt, daß mindestens einer der Reste R³ ein 6-Halogenrest oder ein 6-Alkylrest ist und mindestens einer der Reste R⁴ ein 2-Halogenrest ist.

3. Verbindung nach Anspruch 1, worin die Verbindung ausgewählt ist aus der Gruppe bestehend aus
N-[2,4-Bis-(methylthio)-6-methylpyridin-3-yl]-N'-[6-chlor-4-(2-chlorphenyl)-1,2-dihydro-1-methyl-2-oxochinolin-3-yl]-harnstoff;
N-[4,6-Bis-(methylthio)-2-methylpyrimidin-5-yl]-N'-[6-chlor-4-(2-chlorphenyl)-1,2-dihydro-1-methyl-2-oxochinolin-3-yl-harnstoff;
N-[6-Chlor-4-(2-chlorphenyl)-1,2-dihydro-1-methyl-2-oxochinolin-3-yl]-N'-[6-methylthiochinolin-5-yl)-harnstoff;
N-[6-Chlor-4-(2-chlorphenyl)-1,2-dihydro-1-methyl-2-oxochinolin-3-yl]-N'-(6-methoxychinolin-5-yl)-harnstoff;
N-[4,6-Bis-(methylthio)-2-methylpyrimidin-5-yl)-N'-[4-(2-chlorphenyl)-1,2-dihydro-6-ethyl-1-methyl-2-oxochinolin-3-yl]-harnstoff;
N-[2,4-Bis-(methylthio)-6-methylpyridin-3-yl]-N'-[4-(2-chlorphenyl)-1,2-dihydro-6-ethyl-1-methyl-2-oxochinolin-3-yl]-harnstoff;
N-4-(2-Chlorphenyl)-1,2-dihydro-6-ethyl-1-methyl-2-oxochinolin-3-yl]-N'-(6-methylthiochinolin-5-yl)-harnstoff;
N-[4-(2-Chlorphenyl)-1,2-dihydro-6-ethyl-1-methyl-2-oxochinolin-3-yl]-N'-(6-methoxychinolin-5-yl]-harnstoff;
N-[2,4-Bis-(methylthio)-6-methylpyridin-3-yl]-N'-[4-(2-chlorphenyl)-1,2-dihydro-6-isopropyl-1-methyl-2-oxochinolin-3-yl]-harnstoff;
N-[4,6-Bis-(methylthio)-2-methylpyrimidin-5-yl]-N'-[4-(2-chlorphenyl)-1,2-dihydro-6-isopropyl-1-methyl-2-oxochinolin-3-yl]-harnstoff;
N-[4-(2-Chlorphenyl)-1,2-dihydro-6-isopropyl-1-methyl-2-oxochinolin-3-yl]-N'-(6-methylthiochinolin-5-yl)-harnstoff;
N-[4-(2-Chlorphenyl)-1,2-dihydro-6-isopropyl-1-methyl-2-oxochinolin-3-yl]-N'-(6-methoxychinolin-5-yl)-harnstoff;
N-[2,4-Bis-(methylthio)-6-methylpyridin-3-yl]-N'-[4-(2-chlorphenyl)-1,2-dihydro-1,6-dimethyl-2-oxochinolin-3-yl]-harnstoff;
N-[4-(2-Chlorphenyl)-1,2-dihydro-1,6-dimethyl-2-oxochinolin-3-yl]-N'-(6-methylthiochinolin-5-yl)-harnstoff;
N-[4,6-Bis-(methylthio)-2-methylpyrimidin-5-yl]-N'-[4-(2-chlorphenyl)-1,2-dihydro-1,6-dimethyl-2-oxochinolin-3-yl]-harnstoff;
N-[4-(2-Chlorphenyl)-1,2-dihydro-1,6-dimethyl-2-oxochinolin-3-yl]-N'-(6-methoxychinolin-5-yl)-harnstoff;
N-[6-Chlor-4-(2-chlorphenyl)-1,2-dihydro-1-methyl-2-oxochinolin-3-yl]-N'-(6-methoxyisochinolin-5-yl)-harnstoff.

4. Verbindung nach Anspruch 1, worin Q ausgewählt ist aus der Gruppe bestehend aus 6-Methoxychinolin-5-yl-, 6-Methylthiochinolin-5-yl-, 6-Methoxyisochinolin-5-yl-, 6-Methylthioisochinolin-5-yl-, 2-Methyl-4,6-bis-(methylthio)-pyridin-5-yl-, 6-Methyl-2,4-bis-methylthio)-pyridin-3-yl-, 2,4-Bis-(ethylthio)-pyridin-3-ylresten.

5. Verbindung der Formel 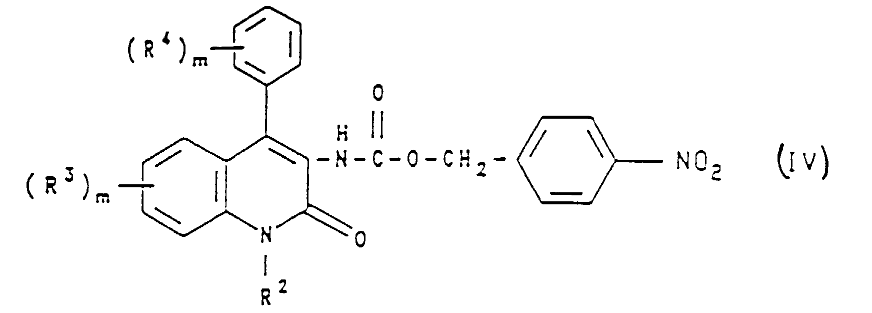 worin m, R², R³ und R⁴ wie in Anspruch 1 definiert sind.

6. Pharmazeutische Zusammensetzung enthaltend eine Verbindung der Formel (I) oder ein pharmazeutisch annehmbares Salz davon nach einem der Ansprüche 1 bis 4 zusammen mit einem pharmazeutisch annehmbaren Verdünnungsmittel oder Träger.

7. Verbindung der Formel (I) und ein pharmazeutisch annehmbares Salz davon nach einem der Ansprüche 1 bis 4 oder eine pharmazeutische Zusammensetzung nach Anspruch 6 zur Verwendung als Arzneimittel.

8. Verwendung einer Verbindung der Formel (I) oder eines pharmazeutisch annehmbaren Salzes davon nach einem der Ansprüche 1 bis 4 oder einer pharmazeutischen Zusammensetzung nach Anspruch 6 zur Herstellung eines Arzneimittels zur Hemmung von Acyl-Coenenzym A:Cholesterinacyltransferase oder der intestinalen Absorption von Cholesterin oder zur Absenkung der Konzentration von Serumcholesterin oder zur Umkehrung oder Verlangsamung der Entwicklung von Atherosklerose.

9. Verfahren zur Herstellung einer Verbindung der Formel I 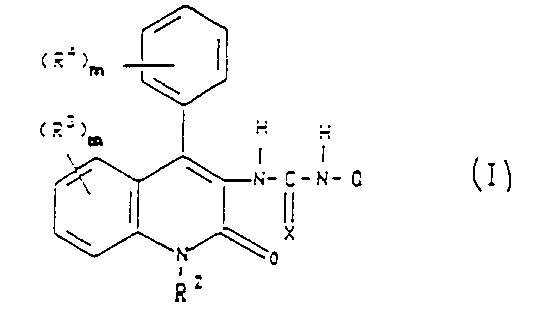 worin R², R³, R⁴, X, m und Q wie in Anspruch 1 definiert sind, das entweder
(a) die Reaktion einer Verbindung der Formel III 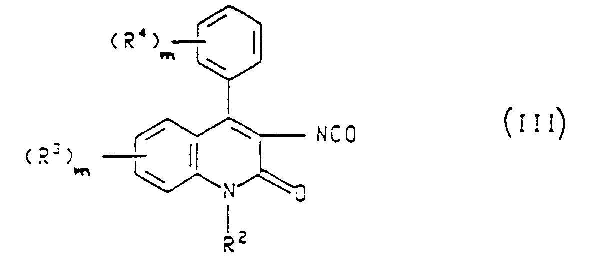 worin R², R³, R⁴ und m wie vorher in diesem Anspruch definiert sind, mit einer Verbindung der Formel QNH2, worin Q wie vorher in diesem Anspruch definiert ist oder
(b) die Reaktion einer Verbindung der Formel V 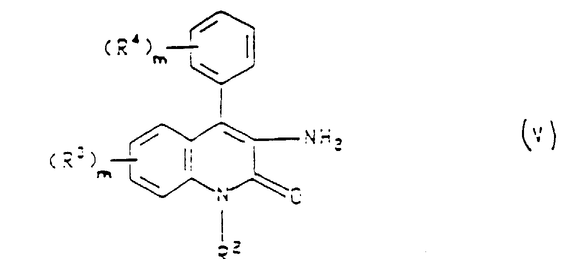 worin R², R³, R⁴ und m wie vorher in diesem Anspruch definiert sind, mit einer Verbindung der Formel QN=C=X, worin Q und X wie vorher in diesem Anspruch definiert sind, umfaßt, woran sich gegebenenfalls die Umwandlung des Produktes in ein pharmazeutisch annehmbares Salz anschließt.

## Revendications

1. Composé de formule 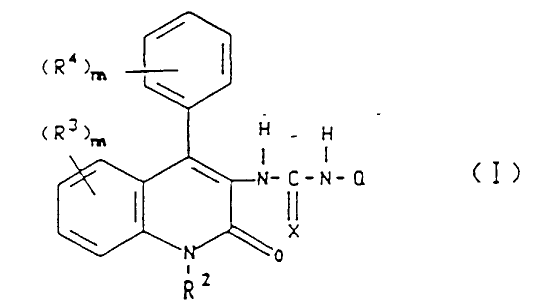 dans laquelle chaque indice m a, indépendamment, une valeur de 0 à 4 ;
R² est choisi entre l'hydrogène et un groupe alkyle en C₁ à C_{6'} et
chacun des groupes R³ et R⁴ est choisi, indépendamment, entre un halogène, un groupe alkyle en C₁ à C₆ facultativement substitué avec un ou plusieurs atomes d'halogènes, un groupe alkoxy en C₁ à C₆ facultativement substitué avec un ou plusieurs atomes d'halogènes, un groupe alkylthio en C₁ à C₆ facultativement substitué avec un ou plusieurs atomes d'halogènes ; un groupe nitro ; un groupe carboxyle facultativement estérifié avec un groupe alkyle en C₁ à C₆ ; un groupe hydroxyle ; un groupe acyloxy en C₁ à C₄ et un groupe acyle en C₁ à C₃ ;
X représente le soufre ou l'oxygène ; et
Q représente un groupe 6-(alkoxy en C₁ à C₃)-quinoléine-5-yle, 6-(alkyle en C₁ à C₃)-thioquinoléine-5-yle, 6-(alkyle en C₁ à C₃)-quinoléine-5-yle, 6-(alkyle en C₁ à C₃)-thio-isoquinoléine-5-yle, 6-(alkoxy en C₁ à C₃)-isoquinoléine-5-yle, 4,6-bis[(alkyle en C₁ à C₃)thio]-2-méthyl-pyrimidine-5-yle, 4,6-bis[(alkyle en C₁ à C₃) thio]-pyrimidine-5-yle, 2,4-bis[(alkyle en C₁ à C₃)thiol]-6-méthyl-pyridine-3-yle ou 2,4-bis[(alkyle en C₁ à C₃)thio]-pyridine-3-yle ; ou un de ses sels pharmaceutiquement acceptables.

2. Composé suivant la revendication 1, dans lequel R² représente un groupe méthyle, m est au moins égal à un et R³ et R⁴ répondent aux définitions précitées, sous réserve qu'au moins un des groupes R³ représente un groupe 6-halogéno ou 6-alkyle et au moins un des groupes R⁴ représente un groupe 2-halogéno.

3. Composé suivant la revendication 1, qui est choisi dans le groupe consistant en les composés suivants
N-[2,4-bis(méthylthio)-6-méthylpyridine-3-yl]-N'-[6-chloro-4-(2-chlorophényl)-1,2-dihydro-1-méthyl-2-oxo-quinoléine-3-yl]urée ;
N-[4,6-bis (méthylthio)-2-méthylpyrimidine-5-yl]-N'-[6-chloro-4-(2-chlorophényl)-1,2-dihydro-1-méthyl-2-oxo-quinoléine-3-yl]urée ;
N-[6-chloro-4-(2-chlorophényl)-1,2-dihydro-1-méthyl-2-oxoquinoléine-3-yl]-N'- (6-méthylthioquinoléine-5-yl)urée ; et
N-[6-chloro-4-(2-chlorophényl)-1,2-dihydro-1-méthyl-2-oxoquinoléine-3 -yl]-N'-(6-méthoxyquinoléine-5-yl)urée ;
N-[4,6-bis(méthylthio)-2-méthylpyrimidine-5-yl]-N'-[4-(2-chlorophényl)-1,2-dihydro-6-éthyl-1-méthyl-2-oxo-quinoléine-3-yl]urée ;
N-[2,4-bis(méthylthio)-6-méthylpyridine-3-yl]-N'-[4-(2-chlorophényl)-1,2-dihydro-6-éthyl-1-méthyl-2-oxo-quinoléine-3-yl]urée ;
N-4-(2-chlorophényl)-1,2-dihydro-6-éthyl-1-méthyl-2-oxoquinoléine-3-yl]-N'-(6-méthylthioquinoléine-5-yl)urée ;
N-[4-(2-chlorophényl)-1,2-dihydro-6-éthyl-1-méthyl-2-oxoquinoléine-3-yl]-Ni-(6-méthoxyquinoléine-5-yl]urée ;
N-[2,4-bis(méthylthio)-6-méthylpyridine-3-yl]-N'-[4-(2-chlorophényl)-1,2-dihydro-6-isopropyl-1-méthyl-2-oxoquinoléine-3-yl]urée ;
N-[4,6-bis(méthylthio)-2-méthylpyrimidine-5-yl]-N'-[4-(2-chlorophényl)-1,2-dihydro-6-isopropyl-1-méthyl-2-oxoquinoléine-3-yl]urée ;
N-[4-(2-chlorophényl)-1,2-dihydro-6-isopropyl-1-méthyl-2-oxoquinoléine-3-yl]-N'-(6-méthylthioquinoléine-5-yl)urée ;
N-[4-(2-chlorophényl)-1,2-dihydro-6-isopropyl-1-méthyl-2-oxoquinoléine-3-yl]-N'-(6-méthoxyquinoléine-5-yl)urée ;
N-[2,4-bis(méthylthio)-6-méthylpyridine-3-yl]-N'-[4-(2-chlorophényl)-1,2-dihydro-1,6-diméthyl-2-oxoquinoléine-3 -yl]urée ;
N-[4-(2-chlorophényl)-1,2-dihydro-1,6-diméthyl-2-oxoquinoléine-3-yl]-N'-(6-méthylthioquino1éine-5-yl)urée ;
N-[4,6-bis(méthylthio)-2-méthylpyrimidine-5-yl]-N'-[4-(2-chlorophényl)-1,2-dihydro-1,6-diméthyl-2-oxo-quinoléine-3-yl]urée ;
N-[4-(2-chlorophényl)-1,2-dihydro-1,6-diméthyl-2-oxoquinoléine-3-yl]-N'-(6-méthoxyquinoléine-5-yl)urée ;
N-[6-chloro-4-(2-chlorophényl)-1,2-dihydro-1-méthyl-2-oxoquinoléine-3-yl]-N'-(6-méthoxyisoquinoléine-5-yl)urée.

4. Composé suivant la revendication 1, dans lequel Q est choisi dans le groupe consistant en les groupes 6-méthoxyquinoléine-5-yle, 6-méthylthioquinoléine-5-yle, 6-méthoxyisoquinoléine-5-yle, 6-méthylthio-isoquinoléine-5-yle, 2-méthyl-4,6-bis(méthylthio)pyrimidine-5-yle, 6-méthyl-2,4-bis(méthylthio)pyridine-3-yle et 2,4-bis(éthylthio)pyridine-3-yle.

5. Composé répondant à la formule 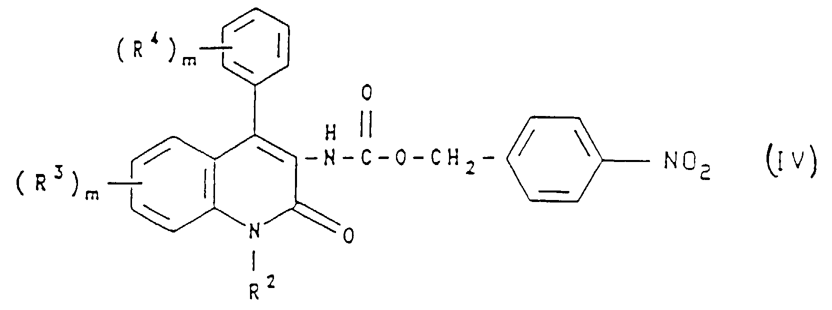 dans laquelle m, R², R³ et R⁴ répondent aux définitions suivant la revendication 1.

6. Composition pharmaceutique comprenant un composé de formule (I) ou un de ses sels pharmaceutiquement acceptables suivant l'une quelconque des revendications 1 à 4, en association avec un diluant ou support pharmaceutiquement acceptable.

7. Composé de formule (I) ou un de ses sels pharmaceutiquement acceptables suivant l'une quelconque des revendications 1 à 4, ou une composition pharmaceutique suivant la revendication 6, destiné à être utilisé comme médicament.

8. Utilisation d'un composé de formule (I) ou d'un de ses sels pharmaceutiquement acceptables suivant l'une quelconque des revendications 1 à 4, ou d'une composition pharmaceutique suivant la revendication 6, pour la production d'un médicament destiné à inhiber l'acyl-coenzyme A : cholestérol-acyltransférase ou l'absorption intestinale du cholestérol, ou destiné à abaisser la concentration de cholestérol sérique, ou à inverser ou ralentir le développement de l'athérosclérose.

9. Procédé pour la préparation d'un composé de formule I 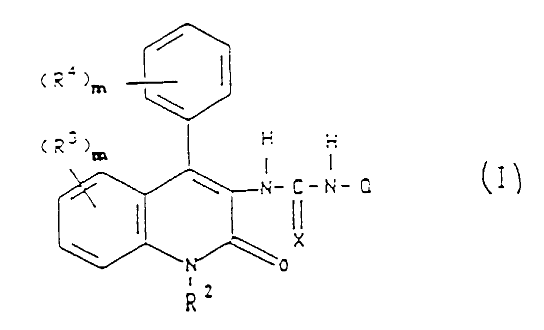 dans laquelle R², R³, R⁴, X, m et Q répondent aux définitions précitées dans la revendication 1, qui comprend soit
(a) la réaction d'un composé de formule III 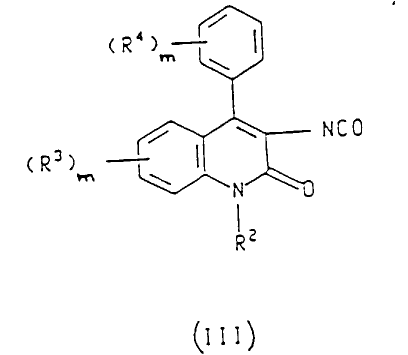 dans laquelle R², R³, R⁴ et m répondent aux définitions mentionnées précédemment dans cette revendication, avec un composé de formule QNH₂, dans laquelle Q répond à la définition mentionnée précédemment dans cette revendication, soit
(b) la réaction d'un composé de formule V 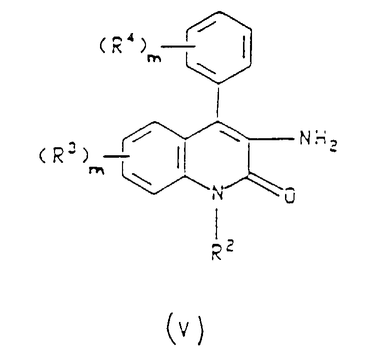 dans laquelle R², R³, R⁴ et m répondent aux définitions mentionnées précédemment dans cette revendication, avec un composé de formule QN=C=X, dans laquelle Q et X répondent aux définitions mentionnées précédemment dans cette revendication, avec ensuite facultativement la transformation du produit en un sel pharmaceutiquement acceptable.
